# EUROPEAN PATENT APPLICATION

(11) **EP 4 650 774 A1**
(43) Date of publication of application: **19.11.2025**
(21) Application number: 24382533.8
(22) Date of filing: 17.05.2024
(51) Int. Cl.: G01N 33/50, C12Q 1/68, G01N 33/68

(54) **HEY2 INHIBITORS FOR USE IN THE TREATMENT OF CARDIAC DISEASES IN MAMMALS**

(71) Applicant: Centro Nacional de Investigaciones Cardiovasculares Carlos III (F.S.P.), 28029 Madrid (ES)
(72) Inventor: Torres Sánchez, Miguel, 28029 Madrid (ES); Villa del Campo, Cristina, 28029 Madrid (ES); Rivero García, Inés, 28029 Madrid (ES)
(74) Representative: Hoffmann Eitle

(57) **Abstract**

The present invention refers to the medical field. Particularly, the present invention refers to methods for the identification and/or production of compounds suitable for the treatment of a cardiac disease in mammals. It also refers to HEY2 inhibitors for use in the treatment of cardiac diseases in mammals.

## Description

### FIELD OF THE INVENTION

The present invention refers to the medical field. Particularly, the present invention refers to methods for the identification and/or production of compounds suitable for the treatment of a cardiac disease in mammals. It also refers to HEY2 inhibitors for use in the treatment of cardiac diseases in mammals.

### STATE OF THE ART

Heart failure is a leading cause of morbidity and mortality worldwide. Although current treatments delay the progression of heart failure and improve survival, ultimately pump replacement using left-ventricular assisted device (LVAD) or heart transplantation is required. An important pathophysiological mechanism that mediates heart failure progression is the lack of spontaneous regenerative capacity of the adult mammalian heart. The newborn hearts of mice and pigs can spontaneously regenerate for the first few days of life, which is mediated by cardiomyocyte (CM) proliferation. This regenerative potential is lost shortly after birth as CMs go through the final round of DNA replication without cytokinesis, becoming polyploid around postnatal day P5-P7 in mouse. Therefore, the myocardial growth mode shifts from hyperplasic to hypertrophic as CMs permanently exit the cell cycle. Although CM turnover exists in adult mammals, the turnover rate is around 0.5% annually, which is insufficient to recover cardiac function after injury. Therefore, upon pathological cardiac overload, the adult heart does not regenerate but rather remodels with further CM hyperploidization and hypertrophy.

The functional reserve of the heart in response to an increased demand thus critically depends on CM hypertrophy, which occurs physiologically during organism growth or exercise training. Fully differentiated ventricular polyploid CMs (PCMs) of the working myocardium are highly specialized cells with a well-structured sarcomere that occupies most of their cytoplasm and are predominantly polyploid (in the mouse, most adult ventricular CMs contain two nuclei). For adult ventricular CMs to divide, sarcomere disassembly is required, resulting in a transient loss of contractile ability. Furthermore, polyploidy and multinucleation provoke mitotic instability, which represents an important barrier for cell proliferation. Despite these limitations, several factors can trigger sarcomere disassembly and engage CMs in proliferation, allowing re-muscularization of the myocardium following massive, localized CM loss after experimental acute myocardial infarction. For these reasons, the small proportion of adult CMs that remain diploid, which are referred to as adult diploid cardiomyocytes (ADCs), have been proposed as a key population that retains proliferative potential and could be stimulated for heart regeneration. In favour of this view, the newborn mammalian heart contains mainly diploid CMs and can regenerate, while polyploidization of CMs around postnatal day 7 coincides with the loss of regenerative ability. In addition, abundance of ADCs in the adult mouse heart strongly varies with the genetic background and a positive correlation has been reported between the abundance of ADCs and cardiac function recovery following a myocardial infarction. The naturally regenerative adult zebrafish heart is composed of diploid CMs that are able to proliferate, but lose this ability if experimentally forced to become polyploid. Despite this, previous efforts to identify a molecular signature for ADCs have been unsuccessful. Moreover, E2F7/8 elimination in mouse CMs increases the proportion of mononuclear CMs but does not improve heart regeneration. As a result, the current view in the field is that mono-nuclear CMs do not necessarily promote cardiac regenerative ability and a second hit would be required. Currently, the inability to identify the molecular signature of ADCs represents an important limitation in the field of cardiac regeneration. At the moment it is not possible to identify, trace or specifically stimulate ADCs for understanding their relevance in heart homeostasis and potential roles in heart regeneration. Here, the inventors developed new approaches for single-CM molecular characterization and identified the ADC molecular signature. This finding places us in a unique position to approach this promising opportunity for mammalian heart regeneration.

So, there is a need for the development of efficient strategies for the treatment of cardiac diseases in mammals. The present invention is focused on solving this problem and methods for the identification and/or production of compounds suitable for the treatment of a cardiac disease in mammals, are herein provided, as well as inhibitors suitable for the treatment of cardiac diseases in mammals.

### DESCRIPTION OF THE INVENTION

### Brief description of the invention

The present invention refers to a genetic *HEY2* inhibitor, or pharmaceutical composition comprising thereof, for use in the treatment of cardiac diseases in mammals. It also refers to methods for the identification and/or production of compounds suitable for the treatment of a cardiac disease in mammals.

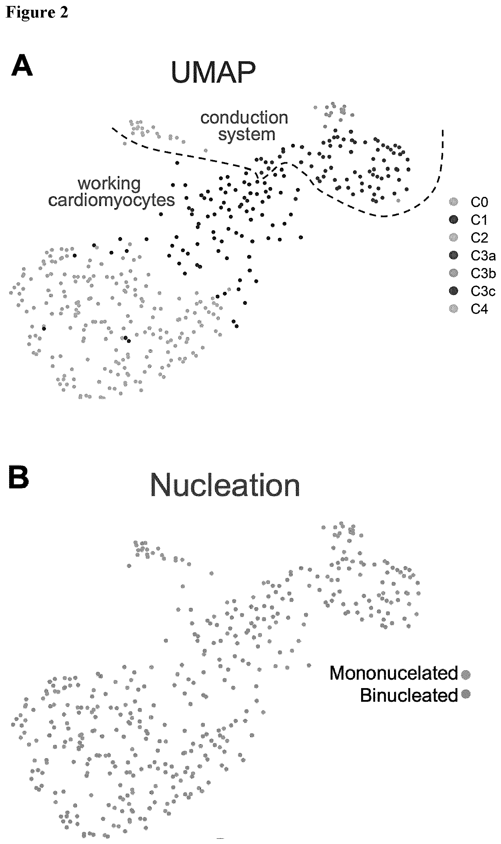

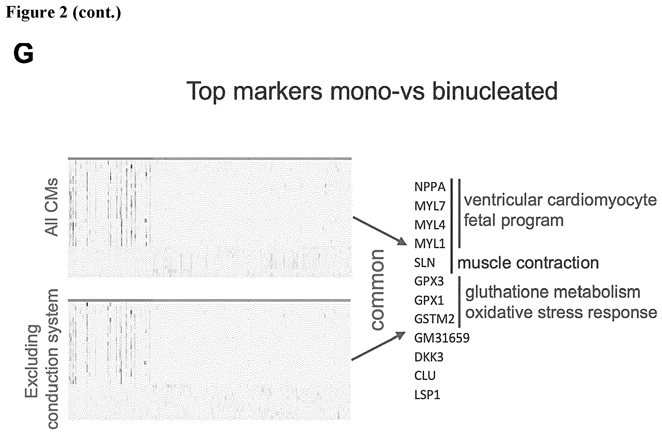

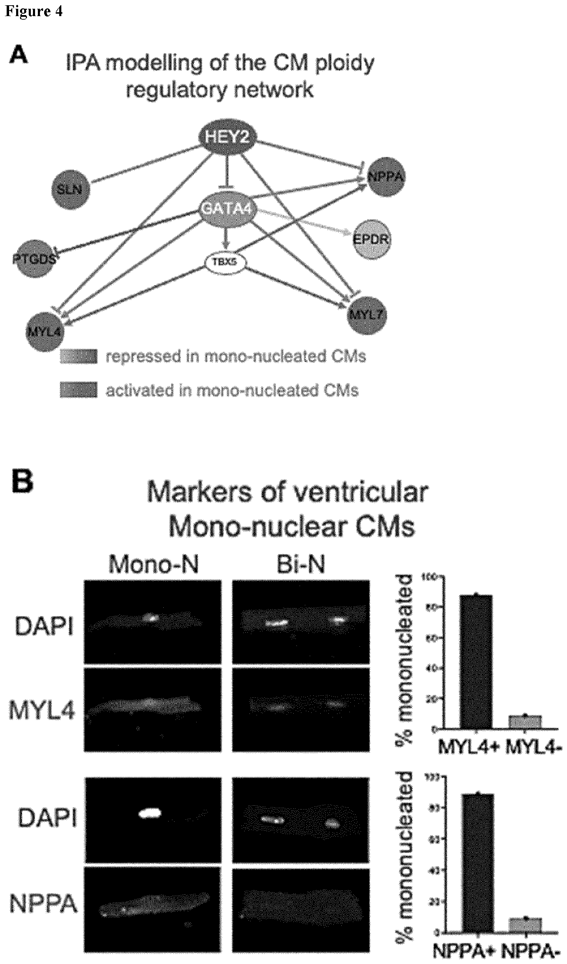

By comparing transcriptomic data from mononucleated and binucleated CMs, the inventors identified a genetic signature of mononucleated CMs **(****Figure 2G****)** that are part of a regulatory transcriptional network regulated by Hey2. These results suggest a role of Hey2 as a marker of binucleated CMs **(****Figure 4A****).**

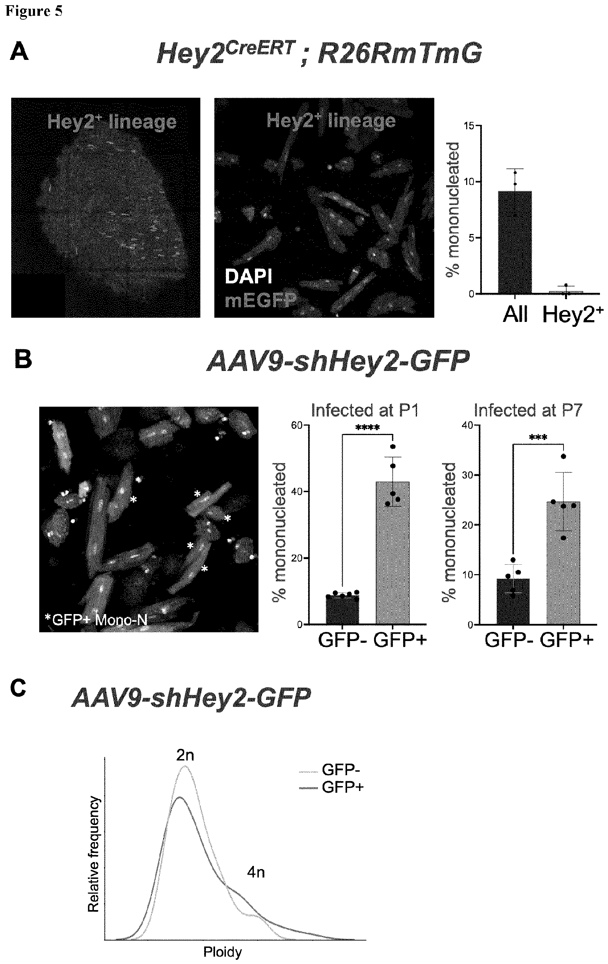

To elucidate whether Hey2 is a marker of binucleated CMs *in vivo,* the inventors used a hey2-CreERT2 mouse line and traced the lineage of Hey2-expressing CMs in adult mice **(****Figure 5A****).** While the lineage-negative CMs matched the general population of ~8% of mononuclear CMs, only one out of the 189 scored mononucleated CMs were belonged to the Hey2 lineage-positive population **(****Figure 5A****).** These results further support a role of Hey2 as a marker of binucleated CMs *in vivo.*

To understand the ability of Hey2 to regulate the adult CM identity, the inventors infected mice with AAV9-Hey2shRNA, which specifically inhibits Hey2 expression in CMs *in vivo.* Infected CMs displayed a notably higher percentage of mononucleated cells compared to uninfected cells **(****Figure 5B****),** even infection was performed during the binucleation process (Figure 9B). These findings suggest that Hey2 inhibition can prevent CM binucleation, which represents a new pro-regenerative cellular mechanism. These results confirm that Hey2 is a master regulator of binucleation in ventricular CMs and that the sole inhibition of its activity is enough to robustly increase the ADC population.

In our opinion, these results provide sufficient proof for the use of genetic *HEY2* inhibitors in the treatment of cardiac diseases in mammals.

A first aspect of the invention refers to a HEY2 inhibitor, or pharmaceutical composition comprising thereof, for use in the treatment of cardiac diseases in mammals.

In a preferred embodiment, the HEY2 inhibitor is selected from:
i) a genetic *HEY2* inhibitor, or
ii) a peptide or polypeptide that interferes with the interaction between HEY2 and other proteins, or a nucleic acid encoding thereof.

In a more preferred embodiment, the peptide or polypeptide interferes with the interaction between HEY2 and HES1 or HES2. These molecules can be administered through different delivery methods, including lipid nanoparticles, cell-penetrating peptides like TAT peptide, or through gene therapy approaches utilizing viral vectors or lipid nanoparticles to introduce nucleic acids encoding inhibitory factors.

In another more preferred embodiment, the genetic *HEY2* inhibitor comprises a nucleic acid directed towards the *HEY2* locus or a *HEY2* transcript.

In another more preferred embodiment, the genetic *HEY2* inhibitor is selected from the group consisting of:
a) an RNA molecule characterized in that it is designed to inhibit *HEY2* expression by interacting with HEY2 RNA molecules through base complementarity thereby preventing them from being translated, preferably selected from the group consisting of a small interfering RNA molecule (siRNA), an anti-sense RNA molecule, a microRNA molecule (miRNA) and/or a short hairpin RNA (shRNA), and/or
b) a genome editing tool characterized in that it is designed to disrupt the *HEY2* locus or the *HEY2* gene thereby altering its expression or function, preferably selected from the group consisting of zinc-finger proteins (ZFNs), transcription activator-like effector nucleases (TALENs) and/or CRISPR/Cas.

In another more preferred embodiment, the genetic *HEY2* inhibitor is an RNA molecule directed towards the *HEY2* locus or a *HEY2* transcript.

In a preferred embodiment, the cardiac disease is selected from: ischemic heart failure, non-ischemic heart failure, heart failure with preserved ejection fraction, idiopathic dilated cardiomyopathy, acute myocardial infarction and/or myocarditis.

In a preferred embodiment, the HEY2 inhibitor, or pharmaceutical composition comprising thereof, is for use in increasing the frequency of adult diploid cardiomyocytes.

In another preferred embodiment, the HEY2 inhibitor, or pharmaceutical composition comprising thereof, is for use in the promotion of cardiac regeneration.

In another preferred embodiment, the HEY2 inhibitor, or pharmaceutical composition comprising thereof, is for use in increasing the frequency of adult diploid cardiomyocytes.

In another preferred embodiment, the HEY2 inhibitor, or pharmaceutical composition comprising thereof, is for use in the promotion of cardiac regeneration.

In another preferred embodiment, the HEY2 inhibitor, or pharmaceutical composition comprising thereof, is for use in the treatment of cardiac diseases in mammals, and the HEY2 inhibitor is a genetic *HEY2* inhibitor selected from the group consisting of:
a) an RNA molecule characterized in that it is designed to inhibit *HEY2* expression by interacting with HEY2 RNA molecules through base complementarity thereby preventing them from being translated, preferably selected from the group consisting of a small interfering RNA molecule (siRNA), an anti-sense RNA molecule, a microRNA molecule (miRNA) and/or a short hairpin RNA (shRNA), and/or
b) a genome editing tool characterized in that it is designed to disrupt the *HEY2* locus or the *HEY2* gene thereby altering its expression or function, preferably selected from the group consisting of zinc-finger proteins (ZFNs), transcription activator-like effector nucleases (TALENs) and/or CRISPR/Cas.
and the cardiac disease is selected from the group consisting of ischemic heart failure, non-ischemic heart failure, heart failure with preserved ejection fraction, idiopathic dilated cardiomyopathy, acute myocardial infarction and/or myocarditis.

A second aspect of the invention refers to a method for the identification and/or production of compounds suitable for the treatment of a cardiac disease in mammals, which comprises the steps of:
a) assessing the expression of the *HEY2* gene once the candidate compound has been incubated with a genetic construct comprising the *HEY2* locus or a fragment thereof, or with cells containing a genetic construct comprising the *HEY2* locus or a fragment thereof,
b) wherein if a reduction in the expression of the *HEY2* gene is observed, it is indicative that the candidate compound may be suitable for the treatment of a cardiac disease.

The cardiac diseases are the same as described for the first aspect. Thus, all the embodiments of the description mentioned in this regard for the first aspect applies herein.

A third aspect of the invention refers to a method for the identification and/or production of compounds suitable for the treatment of a cardiac disease in mammals, which comprises the steps of:
a) assessing the expression of the HEY2 protein, or the activity of the HEY2 protein, once the candidate compound has been incubated with the HEY2 protein, or with cells containing a genetic construct comprising the *HEY2* locus or a fragment thereof,
b) wherein if an inhibition of the HEY2 protein expression or activity is observed, it is indicative that the candidate compound may be suitable for the treatment of a cardiac disease.

The cardiac diseases are the same as described for the first aspect. Thus, all the embodiments of the description mentioned in this regard for the first aspect applies herein.

Each embodiment disclosed herein is contemplated as being applicable to each of the other disclosed embodiments. Thus, all combinations of the various elements described herein are within the scope of the invention.

Unless otherwise stated, the terms used in the present application have the meanings indicated herein. Terms that have not been defined herein should be given the meanings that would be given to them by a person skilled in the art in the context of this disclosure.

**In the context of the present invention the following terms are defined:**
- The term "HEY2 inhibitor" refers to compounds or agents aimed at inhibiting the function of the HEY2 protein. Beyond genetic *HEY2* inhibitors (see definition below), the term "HEY2 inhibitor" also encompasses i) small molecule inhibitors, such as chemical compounds designed to bind specifically to HEY2 and inhibit its activity, ii) antibody-based inhibitors can target HEY2 through specific binding interactions, preventing its function in various cellular processes, and/or iii) polypeptides that interfere with the interactions between HEY2 and other proteins, such as polypeptides designed to prevent the interaction of HEY2 with HES1 or HES2. These molecules can be administered through different delivery methods, including lipid nanoparticles, cell-penetrating peptides like TAT peptide, or through gene therapy approaches utilizing viral vectors or lipid nanoparticles to introduce nucleic acids encoding inhibitory factors. These diverse strategies highlight the versatility and potential of HEY2 inhibition in therapeutic interventions for diseases where HEY2 plays a role. Please note that while the expression "HEY2 inhibitor" includes the nomenclature of the human HEY2 homologue "HEY2" (i.e., in capital letters), the term also intends to include other mammal HEY2 homologues.
- The term "genetic *HEY2* inhibitor" refers to agents, whether natural or synthetic, or strategies that inhibit the expression or activity of the *HEY2* gene through genetic manipulation. This term encompasses various genetic elements, such as RNA interference (RNAi) constructs, CRISPR/Cas9-mediated knockout systems, or other gene editing techniques, designed to disrupt *HEY2* expression. These approaches target the gene encoding HEY2 directly to reduce its expression or disrupt its function, ultimately leading to inhibition of HEY2 activity. Please note that while the expression "*HEY2* inhibitor" includes the nomenclature of the human *HEY2* homologue *"HEY2"* (i.e., in capital letters), the term also intends to include other mammal *HEY2* homologues.
- The term "pharmaceutical composition" refers to a formulation comprising one or more active pharmaceutical ingredients (APIs) along with various excipients, carriers, stabilizers, and other ingredients. These compositions are designed to be administered to patients for therapeutic or diagnostic purposes. They can take various forms such as tablets, capsules, injections, creams, ointments, solutions, suspensions, or patches. The composition is specifically formulated to deliver the active ingredient(s) in a safe, effective, and stable manner, often following regulatory guidelines for pharmaceutical development and manufacturing.
- The term "nucleic acid directed towards the *HEY2* locus or a *HEY2* transcript" refers to a nucleic acid sequence designed to interact and modulate the expression or function of the *HEY2* gene. This can include various types of nucleic acids, such as RNA molecules (e.g. microRNAs, siRNAs, antisense RNAs, guide RNAs), DNA molecules (e.g. gene editing constructs), or synthetic oligonucleotides. In the context of CRISPR/Cas technology, it may specifically denote a CRISPR/Cas cassette containing guide RNAs designed to target the *HEY2* gene locus for disruption, thereby altering its expression or function.
- The term "disrupt the *HEY2* locus or the *HEY2* gene" refers to introducing modifications or changes to the specific genomic location of the *HEY2* gene *(HEY2* locus) or to the *HEY2* gene. This disruption can involve various methods, such as gene editing techniques like CRISPR/Cas, which can delete, insert, or alter nucleotides at the targeted locus. By disrupting the locus, the normal function or expression of the gene located there can be modified or interrupted. In the context of disrupting the *HEY2* locus, it means altering the genomic region where the *HEY2* gene is located, potentially affecting its expression or function.
- The term "cardiac disease", also known as "heart disease" or "cardiovascular disease", refers to a range of conditions that affect the heart and blood vessels. These conditions can include coronary artery disease, heart rhythm disorders (arrhythmias), heart valve problems, heart failure, congenital heart defects, and others. Cardiac diseases can lead to symptoms such as chest pain, shortness of breath, fatigue, dizziness, and swelling in the legs, ankles, or abdomen. They are often caused by risk factors like high blood pressure, high cholesterol, smoking, diabetes, obesity, and lack of physical activity.
- The term "compounds" refers to substances formed by the chemical combination of two or more elements fixed in proportions. These elements are typically bonded together through chemical bonds, resulting in a distinct molecular structure with unique properties. Compounds can range from simple molecules like water (H₂O) to complex organic compounds like proteins and pharmaceutical drugs. They can exhibit a wide range of physical and chemical properties, making them essential in various fields such as medicine, agriculture, material sciences, and more.
- The term "suitable for the treatment of" refers to the capability of a substance, such as a drug or therapy, to effectively address a particular medical condition or disease. It indicates that the substance has demonstrated efficacy, safety, and appropriate pharmacological properties for managing or alleviating the symptoms, progression, or underlying causes of the specific condition. This determination is often based on preclinical and clinical studies, regulatory approvals, and medical guidelines.
- The term *"HEY2* gene" does not intend to refer exclusively to the human *HEY2* gene. Please note that while the expression *"HEY2* gene" includes the nomenclature of the human *HEY2* homologue *"HEY2"* (i.e., in capital letters), the term also intends to include other mammal *HEY2* homologues.
- The term *"HEY2* locus" refers to the specific chromosomal location where the *HEY2* gene is located within the genome. In humans, the *HEY2* gene is found on chromosome 6p12.3. The term "locus" denotes a fixed position on a chromosome where a particular gene or DNA sequence is located. Thus, the *HEY2* locus specifies the precise genomic regions where the *HEY2* gene is situated. Please note that while the expression *"HEY2* locus" includes the nomenclature of the human *HEY2* homologue *"HEY2"* (i.e., in capital letters), the term also intends to include other mammal *HEY2* homologues.
- The term "reduction in the expression" refers to a decrease or lowering of the level of gene expression, typically at the mRNA or protein level. This reduction can occur through various mechanisms, such as transcriptional repression, mRNA degradation, or translational inhibition. It results in fewer copies of the mRNA transcript being produced or fewer functional protein products being synthesized, leading to diminished activity or function of the gene protein.
- The term "HEY2 protein" does not intend to refer exclusively to the human HEY2 protein. It also intends to include other mammal HEY2 homologues.
- The term "comprising" means including, but it is not limited to, whatever follows the word "comprising". Thus, use of the term "comprising" indicates that the listed elements are required or mandatory, but that other elements are optional and may or may not be present.
- The term "consisting of" means including, and it is limited to, whatever follows the phrase "consisting of". Thus, the phrase "consisting of" indicates that the listed elements are required or mandatory, and that no other elements may be present.

### Description of the figures

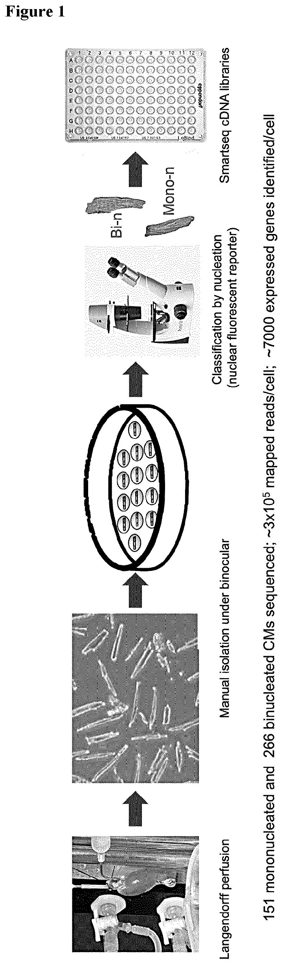

**Figure 1****.** Manual isolation, determination of nucleation and deep RNAseq sequencing of single ventricular CMs.

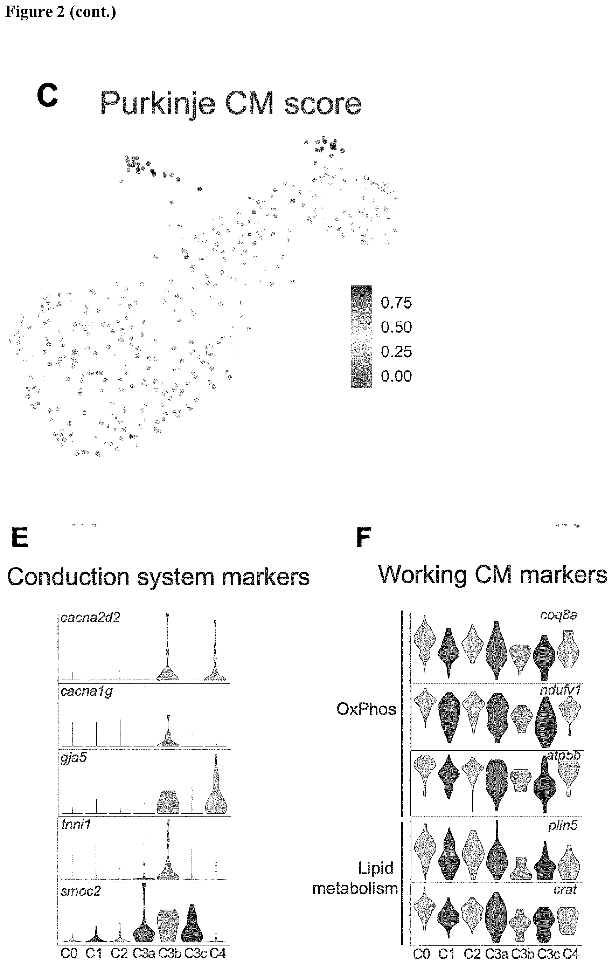

**Figure 2****.** Single-CM RNAseq results. A) UMAP clustering of the ventricular CMs. B) identification of mononuclear and binuclear CMs in the UMAP. C) mapping of a Purkinj e score identifies two CM clusters mostly composed of mononucleated CMs. E) Distribution by cluster of conduction system markers. F) Distribution by cluster of working CM markers. G) Analysis of the markers of mononucleated CMs.

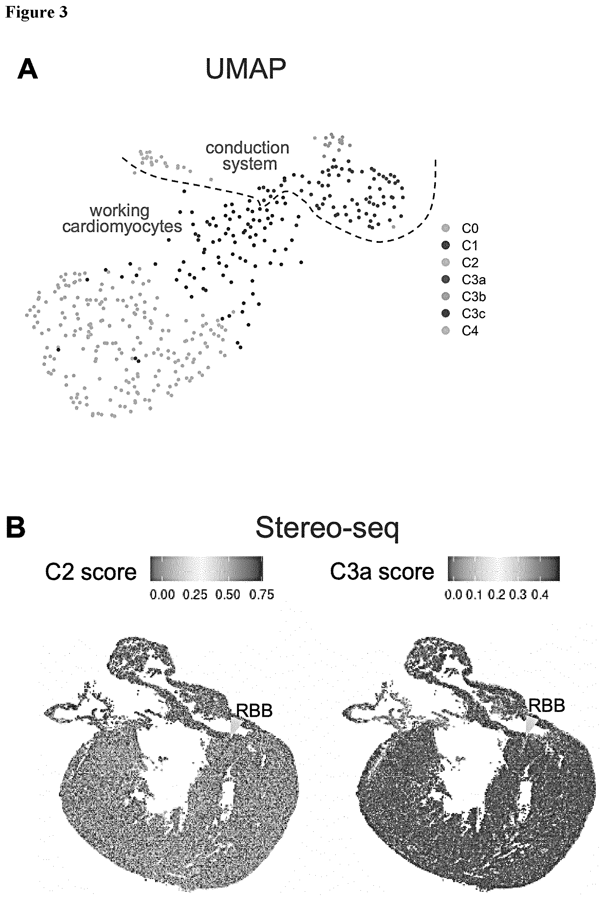

**Figure 3****.** Stereoseq mapping of single-CM RNAseq clusters.

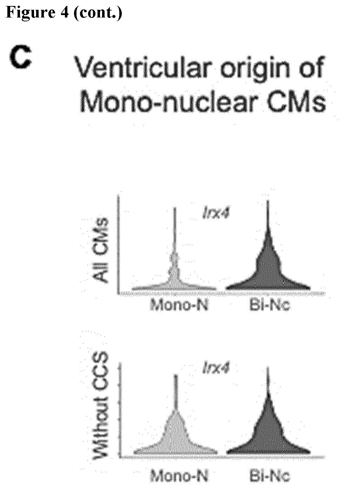

**Figure 4****.** Pathway and marker analysis of Adult Diploid CMs. A) modelling of the regulatory network deduced from the differentially expressed genes between mono- and binucleated CMs. B) confirmation of marker association with mononucleated CMs. C) Confirmation of the ventricular origin of ADCs by the expression pattern of Irx4.

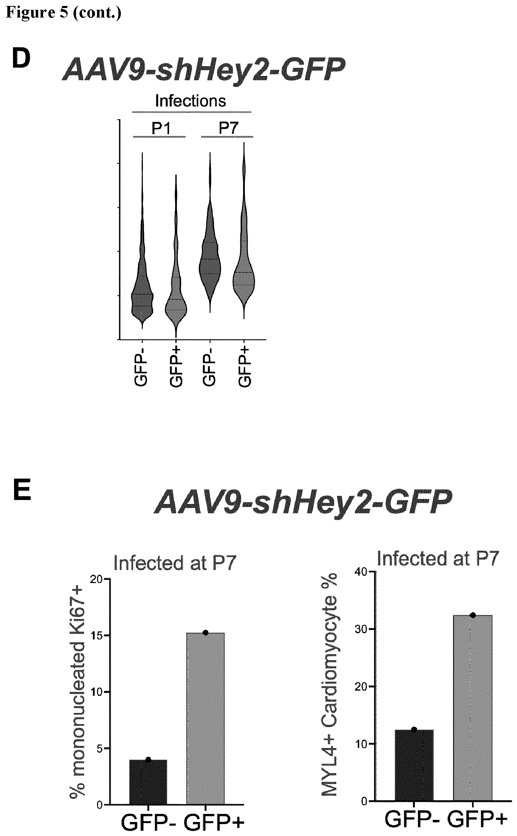

**Figure 5****.** Functional evidence for a role of hey2 in promoting working CMs polyploidy. A) lineage tracing of Hey2-expressing CMs in the adult myocardium. B) Effect of hey2 inhibition in vivo on CM nucleation, ploidy and marker expression. C) Determination of ploidy by measurement of DAPI signal in hey2 knock-down (GFP+) and control (GFP-) Langendorff-isolated cardiomyocytes from the same adult heart by confocal microscopy and image analysis. D). Determination of cell size in hey2 knock-down (GFP+) and control (GFP-) Langendorff-isolated cardiomyocytes from the same adult heart by confocal microscopy and image analysis. E) Determination of the percentage of cells expressing the cell cycle marker Ki67 (left) and the diploid marker MYL4 (right) in hey2 knock-down (GFP+) and control (GFP-) Langendorff-isolated cardiomyocytes from the same adult heart by confocal microscopy and image analysis.

### Detailed description of the invention

The present invention is illustrated by means of the Examples set below without the intention of limiting its scope of protection.

### Example 1. Material and Methods

### Differential expression analysis

Ventricular cardiomyocytes were manually labeled as mononucleated or polynucleated. The differentially expressed genes between the two groups were calculated using the FindMarkers function of the Seurat R package, using MAST as a differential expression analysis method and Log2-normalized counts-per-10⁴ as input. All genes that were detected in at least 10% of the cells from at least one of the populations were tested for differential expression. The p-values were then adjusted by FDR18 to correct for multiple testing.

### Identification of Hey2 as an upstream regulator

The QUIAGEN Ingenuity Pathway Analysis was run on the 112 differentially expressed genes using the core analysis modality with default settings.

### Example 2. Results

The inventors aimed for a full characterization of ADCs in the mouse model. As mentioned, previous efforts to identify a molecular signature for ADCs were unsuccessful. Nonetheless, the inventors noticed inconsistencies in the published results. On one side, single-cell RNAseq of adult ventricular CMs failed to identify cardiac conduction system (CCS) CMs. Recently, Purkinje CMs have been identified as enriched in mono-nuclear CMs, therefore, a single-CM analysis should have identified this population and markers of Purkinj e CMs as enriched in the mono-nuclear fraction, but it did not. Similarly, a bulk RNAseq analysis of FACS-sorted mono-versus bi-nucleated CMs failed to identify a Purkinje CM signature in the mononuclear CM fraction and only 3 genes were identified as differentially expressed between ADC and adult binucleated CMs. The inventors reasoned that, in order to conclude about the molecular profile of ADCs, an improved approach for their identification and single-cell analysis should be develop. They then explored different conditions and identified FACS-sorting and the use of fluorescent DNA intercalating agents as the main sources for poor single-CM sequencing quality. Therefore, they established a methodology for the visual identification of nucleation in isolated CMs expressing a nuclear GFP under a wide-field epifluorescence microscope and a technique for the manual isolation and immediate processing of the cells **(****Figure** 1). This procedure allowed them to obtain a high-quality, high-depth mRNA sequencing of 151 mononucleated and 266 binucleated adult ventricular CMs. Unsupervised clustering of the sequenced CMs accordingly to their expression profile readily identified two clusters with 94% mononuclear CMs **(****Figure 2A**, B). A Purkinje CM transcriptomic signature enrichment algorithm identified the two mononuclear CM clusters as the Purkinje CM population **(****Figure 2C****).** Two additional clusters were identified as CCS, according to known markers **(****Figure 2A****,** E). The data obtained allowed as well to map the signature of the clusters obtained to spatial transcriptomics maps obtained by Stereo-seq, which provides a high resolution (down to 2µm) description of transcripts in histological sections. For example, cluster 2, which corresponds to PCMs, maps in a widespread manner to the right and left ventricles but spares the right bundle branch (RBB), belonging to the CCS **(****Figure 3****).** In contrast, cluster C3a, which corresponds to the CCS, maps in a restricted manner and illuminates the RBB **(****Figure 3****).**

In total, 40% of all mononucleated CMs belong to the conduction system and around half of these are Purkinje CMs. The Purkinje clusters show a very specific molecular signature; however, it is not possible to discriminate whether the signature is due to their mononuclearity or to their identity as Purkinje CMs. Besides, recent results indicate that Purkinje CMs do not play a specific role in cardiac regeneration. The inventors therefore focused their attention on the mononuclear non-CCS CMs, which account for 60% of the total mononuclear population. In this case, mononuclear CMs appear intermingled with binuclear CMs in 3 clusters that they identify as working myocardium, according to their sarcomeric signature and the abundance of fatty acid metabolism enzymes and OxPhos factors **(****Figure 2A**, F). They first identified differentially expressed genes between all mononuclear and binuclear CMs, identifying 85 genes with preferential expression in mononuclear CMs. Then, they did the same procedure excluding the CCS CMs and identified 25 genes with higher expression in mononuclear CMs. The inventors obtained a final list of 12 genes that overlapped between the two lists, which they considered the signature of the mononucleated CMs irrespective of their cluster identity **(****Figure 2G****).** Within these genes, they found markers of CM fetal program, factors of the Gluthatione metabolism involved in ROS degradation, and factors that reduce contractility and calcium cycling **(****Figure 2G****).** This analysis thus identified non-CCS mononuclear CMs as a population that maintains fetal properties and has not fully differentiated as working CMs. Given that the vast majority of adult mononucleated CMs are diploid in homeostatic conditions, this signature can be attributed to ADCs. Interestingly, they did not detect cell cycle markers in the ADC signature, which suggests that ADCs are mostly quiescent in the uninjured adult heart.

Next, the inventors looked for transcription factors predicted to regulate the mononuclear CM signature. They identified with a high score the transcription factor Hey2 as a repressor and GATA4 as an activator of ADC gene expression signature **(****Figure 4A****).** The genes present in ADCs and putatively repressed by Hey2 included Sarcolipin, MYL4, MYI,7 and NPPA. Some of these markers were validated by immunofluorescence on Langendorff-isolated ventricular CMs (Figure 4B).

Hey2 is a transcriptional repressor of the bHLH family. While in previous studies, Hey2 function in CMs has been interpreted as repressing atrial fate, here they isolated exclusively ventricular CMs and yet identified a Hey2-negative gene signature as present in ventricular ADCs. From the relevant markers, at least MYL4, MYI,7 and NPPA are expressed in fetal ventricular CMs until late gestation. Therefore, Hey2 appears to control the transition between ventricular more immature ADCs and more differentiated PCMs, rather than determination of atrial versus ventricular fate. In agreement with this interpretation, ADCs express IRX4, a ventricular-specific marker **(****Figure 4C****)** and studies on the differentiation of human IPSCs in vitro have identified Hey2 as a CM maturation factor. These results call for a reinterpretation of Hey2 role in ventricular CMs. Previous work showed that permanent deletion of hey2 in CMs led to cardiomyopathy, which was attributed to the transformation of ventricular CMs into atrial CMs, unable to provide ventricular function. Their results show that these data can be reinterpreted as CMs retaining their immature but ventricular character, which disables them for providing the postnatal ventricular contraction demands. In support of this idea, sustained reprogramming of CMs by the Yamanaka factors, YAP activation, miRNAs or Erbb2 activation leads to cardiomyopathy. To determine whether indeed Hey2 is a marker of binucleated CMs *in vivo,* they used a hey2-CreERT2 mouse line and traced the lineage of Hey2-expressing CMs in adult mice **(****Figure 5A****).** The labelling was done at low recombination rates, so that the lineage-negative CMs matched the general population with ~8% of mononuclear CMs. In contrast, in the Hey2 lineage-positive population, they only found 1 mononucleated CM out of 189 scored **(****Figure 5A****).** Next, to understand the ability of Hey2 to regulate CM ploidy, they infected mice with AAV9-Hey2shRNA, which specifically inhibits Hey2 expression in CMs *in vivo.* When infected at P1 and analysed at P22, among the infected CMs, the mononucleated fraction was 42%, whereas it remained at around 8% in uninfected CMs of the same heart **(****Figure 5B****).** Strikingly, when infected at P7 and analysed at P28, they still observed an increase from 8 to 25% of mononucleated CMs in the infected population, despite Hey2 inhibition in this case taking place during the binucleation window (Figure 9B). These findings suggest that Hey2 inhibition can prevent CM binucleation, which would represent a new pro-regenerative cellular mechanism. These results confirm that Hey2 is a master regulator of binucleation in ventricular CMs and that the sole inhibition of its activity is enough to robustly increase the ADC population. Furthermore, the increased population of mononuclear CMs following Hey2 repression remained mostly diploid **(****Figure 5C****),** showed a smaller size **(****Figure 5D****)** and increased proliferation markers and ADC markers **(****Figure 5E****).** These finding suggests that the fully differentiated hypertrophic state of adult ventricular CMs requires Hey2 expression. In fact, Hey2 is permanently expressed in adult working ventricular CMs, however, the way Hey2 is regulated, its interaction partners and its targets in adult CMs remain unknown.

## Claims

1. A method for the identification and/or production of compounds suitable for the treatment of a cardiac disease in mammals, which comprises the steps of:
i) assessing the expression of the *HEY2* gene once the candidate compound has been incubated with a genetic construct comprising the *HEY2* locus or a fragment thereof, or with cells containing a genetic construct comprising the *HEY2* locus or a fragment thereof,
ii) wherein if a reduction in the expression of the *HEY2* gene is observed, it is indicative that the candidate compound may be suitable for the treatment of a cardiac disease.

2. A method for the identification and/or production of compounds suitable for the treatment of a cardiac disease in mammals, which comprises the steps of:
i) assessing the expression of the HEY2 protein, or the activity of the HEY2 protein, once the candidate compound has been incubated with the HEY2 protein, or with cells containing a genetic construct comprising the *HEY2* locus or a fragment thereof,
ii) wherein if an inhibition of the HEY2 protein expression or activity is observed, it is indicative that the candidate compound may be suitable for the treatment of a cardiac disease.

3. The method, according to any of the claims 1 or 2, wherein the cardiac disease is selected from the group consisting of ischemic heart failure, non-ischemic heart failure, heart failure with preserved ejection fraction, idiopathic dilated cardiomyopathy, acute myocardial infarction and/or myocarditis.

4. A HEY2 inhibitor, or pharmaceutical composition comprising thereof, for use in the treatment of cardiac diseases in mammals.

5. The HEY2 inhibitor, or pharmaceutical composition comprising thereof, for use, according to claim 4, wherein the HEY2 inhibitor is selected from:
i) a genetic *HEY2* inhibitor, or
ii) a peptide or polypeptide that interferes with the interaction between HEY2 and other proteins, or a nucleic acid encoding thereof.

6. The HEY2 inhibitor, or pharmaceutical composition comprising thereof, for use, according to claim 5, wherein the genetic *HEY2* inhibitor comprises a nucleic acid directed towards the *HEY2* locus or a *HEY2* transcript.

7. The HEY2 inhibitor, or pharmaceutical composition comprising thereof, for use, according to any of the claims 5 or 6, wherein the genetic *HEY2* inhibitor is selected from the group consisting of:
i) an RNA molecule **characterized in that** it is designed to inhibit *HEY2* expression by interacting with HEY2 RNA molecules through base complementarity thereby preventing them from being translated, preferably selected from the group consisting of a small interfering RNA molecule (siRNA), an anti-sense RNA molecule, a microRNA molecule (miRNA) and/or a short hairpin RNA (shRNA), and/or
ii) a genome editing tool **characterized in that** it is designed to disrupt the *HEY2* locus or the *HEY2* gene thereby altering its expression or function, preferably selected from the group consisting of zinc-finger proteins (ZFNs), transcription activator-like effector nucleases (TALENs) and/or CRISPR/Cas.

8. The HEY2 inhibitor, or pharmaceutical composition comprising thereof, for use, according to any of the claims 5 to 7, wherein the genetic *HEY2* inhibitor is an RNA molecule directed towards the *HEY2* locus or a *HEY2* transcript.

9. The HEY2 inhibitor, or pharmaceutical composition comprising thereof, for use, according to any of the claims 4 to 8, wherein the cardiac disease is selected from the group consisting of ischemic heart failure, non-ischemic heart failure, heart failure with preserved ejection fraction, idiopathic dilated cardiomyopathy, acute myocardial infarction and/or myocarditis.

10. The HEY2 inhibitor, or pharmaceutical composition comprising thereof, for use, according to any of the claims 4 to 9, in increasing the frequency of adult diploid cardiomyocytes.

11. The HEY2 inhibitor, or pharmaceutical composition comprising thereof, for use, according to any of the claims 4 to 10, in the promotion of cardiac regeneration.

12. The HEY2 inhibitor, or pharmaceutical composition comprising thereof, for use, according to any of the claims 5, 7 or 9, in the treatment of cardiac diseases in mammals, wherein the HEY2 inhibitor is a genetic *HEY2* inhibitor selected from the group consisting of:
i) an RNA molecule **characterized in that** it is designed to inhibit *HEY2* expression by interacting with HEY2 RNA molecules through base complementarity thereby preventing them from being translated, preferably selected from the group consisting of a small interfering RNA molecule (siRNA), an anti-sense RNA molecule, a microRNA molecule (miRNA) and/or a short hairpin RNA (shRNA), and/or
ii) a genome editing tool **characterized in that** it is designed to disrupt the *HEY2* locus or the *HEY2* gene thereby altering its expression or function, preferably selected from the group consisting of zinc-finger proteins (ZFNs), transcription activator-like effector nucleases (TALENs) and/or CRISPR/Cas,
and wherein the cardiac disease is selected from the group consisting of ischemic heart failure, non-ischemic heart failure, heart failure with preserved ejection fraction, idiopathic dilated cardiomyopathy, acute myocardial infarction and/or myocarditis.

13. The HEY2 inhibitor, or pharmaceutical composition comprising thereof, for use, according to claims 12, wherein the genetic *HEY2* inhibitor is an RNA molecule directed towards the *HEY2* locus or a *HEY2* transcript.

14. The HEY2 inhibitor, or pharmaceutical composition comprising thereof, for use, according to claim 12, in increasing the frequency of adult diploid cardiomyocytes.

15. The HEY2 inhibitor, or pharmaceutical composition comprising thereof, for use, according to claim 12, in the promotion of cardiac regeneration.
